# EUROPEAN PATENT APPLICATION

(11) **EP 4 339 921 A1**
(43) Date of publication of application: **20.03.2024**
(21) Application number: 23197422.1
(22) Date of filing: 14.09.2023
(51) Int. Cl.: G09B 19/00, A63B 24/00, A63B 71/06

(54) **METHOD FOR THE AUTOMATED GENERATION OF TRAINING SESSIONS TO BE PERFORMED USING EXERCISE MACHINES, AND RELATIVE COMPUTER PROGRAM PRODUCT**

(30) Priority: 15.09.2022 IT 202200018879
(71) Applicant: Technogym S.p.A., 47521 Cesena, Forli'-Cesena (IT)
(72) Inventor: DOMENICHINI, Andrea, 47521 CESENA (IT); TURI, Pietro, 47521 CESENA (IT); MONTANARI, Filippo, 47521 CESENA (IT)
(74) Representative: Lavoix

(57) **Abstract**

Method (100) for generating at least one training session (2) to be performed using at least one exercise machine, characterized in that it comprises at least one phase (120) in which, starting from a digital representation of a waveform (1) which is representative of a chosen audio track and is constituted by a time sequence of discrete reference values (I_{M}), there is generated said at least one training session (2) formed of one or more blocks (10, 30, 70) each block consisting in turn of one or more sub-blocks (12, 14, 15,. ..; 31, 32,...60; 71, 72), wherein each sub-block is defined as a function of at least one parameter related to one or more of said discrete reference values (I_{M}) of the waveform (1) and of a reference physiological parameter related to a user or type of users who has/have to perform said at least one training session (2).

## Description

The present invention relates in general to the field of fitness.

In particular, the present invention relates to a method for the automated generation of training sessions to be performed using exercise machines, and to a related computer program product.

The method and computer program product according to the invention are particularly suitable for the generation of training sessions to be performed using bicycles commonly utilized in training/fitness centres, such as gyms, clubs, etc., for example the so-called stationary bikes, indoor cycles and similar and, therefore, in the following description they will be referred to as examples of exercise machines.

Clearly, and as will become evident from the following description, said reference must be understood solely for the purpose of an exemplary but non-limiting embodiment, since the training sessions generated thanks to the method and computer program product according to the invention can be performed, depending on the requirements and wishes of the users, utilizing other types of exercise machines, such as for example treadmills, rowing machines, elliptical machines, etc.

As is known, in various fitness centres, aerobic/anaerobic training sessions are organised for groups of people who use for example appropriate bicycles.

Depending on the specific requirements, said sessions are of a certain duration, for example about one hour, and are composed of one or more blocks, each block consisting in turn of one or more sub-blocks or steps..

For example, a typical training session performed in the gym using bicycles, foresees an initial warm-up block which comprises for example two steps with diverse and increasing intensities, a central block usually with substantially increased intensity such as for example a fartlek block where high intensity steps are alternated with brief recovery periods, and a final block where several steps are performed with diverse and decreasing intensities until conclusion of the session.

Often during said sessions, music is transmitted so as to make the atmosphere more appealing and the training more enjoyable.

At present, the creation of said training sessions is carried out by trainers who manually compose the profile of each specific session, for example by inserting various data into a computer and/or tables, so gradually putting together the various planned blocks and steps.

Evidently, this system is not optimal since it requires a considerable amount of time; this then becomes even more complex and lengthy if, within the same group, a trainer must further calibrate the sessions and its various blocks on the basis of the requirements and/or performance abilities of one or more participants in the training group.

The aim of the present invention is to address and mitigate at least such issue, and in particular to offer a solution which makes it possible to optimize and speed up the definition of the training sessions compared to the methods used at present.

This aim, as well as others which will become more apparent from the following description, is reached by a method for the generation of training sessions to be performed using exercise machines whose characteristics are defined in claim 1.

This aim, as well as others which will become more apparent from the following description, is reached by a computer program according to claim 14.

Particular embodiments are object of the respective dependent claims whose content is intended as an integral part of the present description.

Further characteristics and advantages of the invention will become more apparent from the following detailed description of exemplary and non-limiting embodiments, and with reference to the attached drawings in which:
figure 1 is a flow diagram schematically illustrating a possible embodiment of a method for the generation of training sessions to be performed using exercise machines according to the invention;
figure 2 is a view illustrating an example of a digital representation of a waveform, relative to an audio track, which can be generated or utilized with the method according to the invention;
figure 3 is a view illustrating a training session profile for bicycles generated by the method according to the invention, for example on the basis of the waveform in figure 2.

It should be noted that in the following detailed description, identical or similar components, from both structural and/or functional points of view, may be indicated with the same or different reference numbers, independently of the fact that they may be shown or described in different embodiments of the present invention or in distinct parts.

It should also be noted that, in order to illustrate this invention clearly and concisely, the drawings are not necessarily to scale and certain characteristics of the description may be shown in a rather schematic form.

Furthermore, when the term "configured" or a similar term is used in this document with reference for example to electronic hardware or software means/devices, said term is intended in the sense that the corresponding means/device includes electronic circuits, or parts of circuits, and/or software/hardware, such as for example algorithms, routines and programs in general, necessary for the execution of the planned functions/steps.

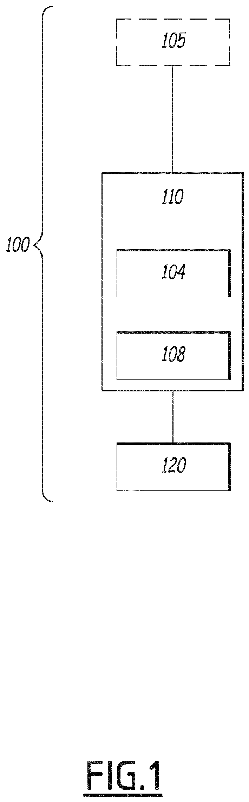

Figure 1 schematically illustrates a method according to the present invention for the generation of at least one training session to be performed using exercise machines, such as bicycles or indoor cycles, indicated by the overall reference number 100.

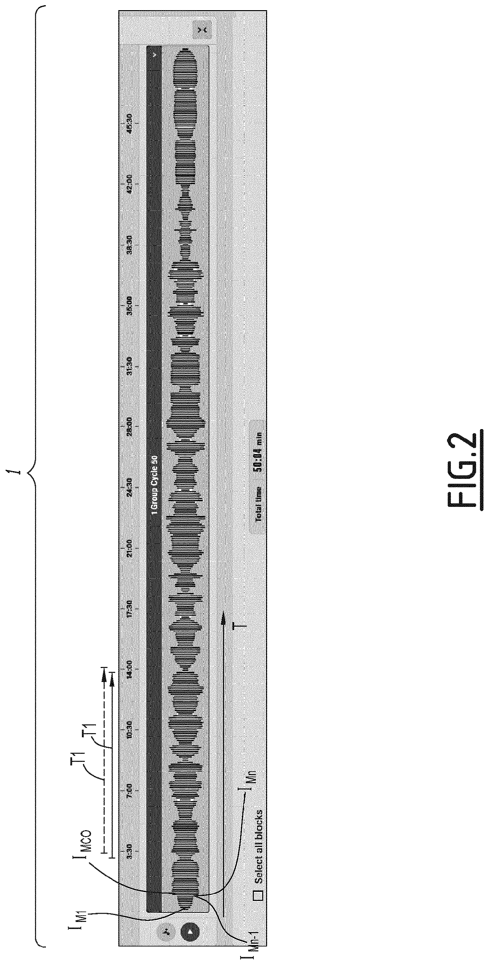

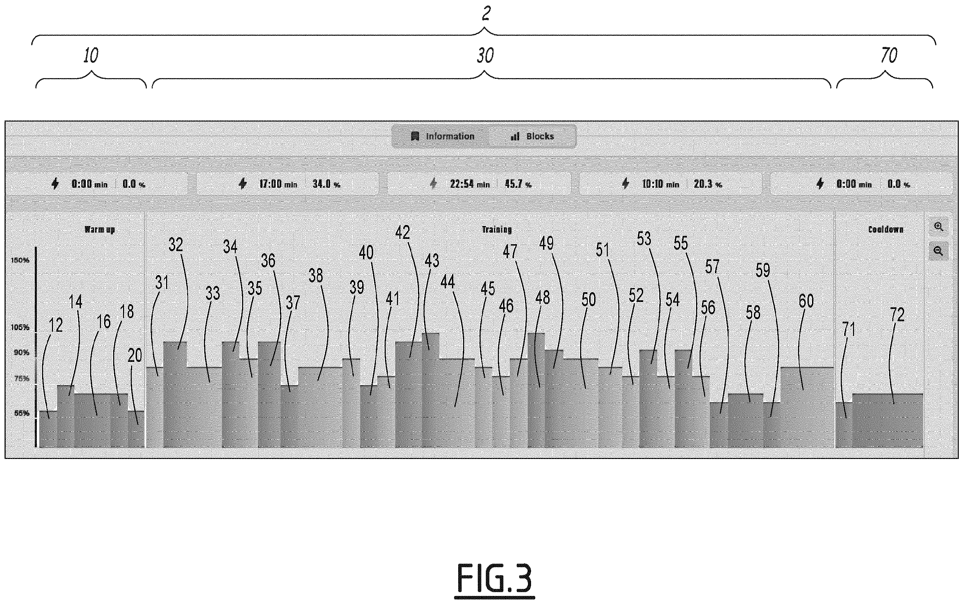

In particular, the method 100 according to the invention comprises at least a first phase 120 in which, starting from a digital representation of a waveform which is representative of a chosen audio track and is constituted by a time sequence of discrete reference values I_{M}, for example the waveform represented in figure 2 by the reference number 1, at least one training session is generated, substantially automatically, by means of an electronic processing unit (not illustrated in the figure), an example of which is indicated in figure 3 by the overall reference number 2.

Within the scope of the present invention, the term electronic processing unit is to be intended in the broadest sense possible and includes any suitable type of electronic device having data processing capacity, such as a processor, for example a digital signal processor (DSP), a microcontroller, etc., and which can further comprise or be operatively associated to circuit means and/or to one or more software modules necessary to carry out the functions/phases foreseen in the method 100.

In particular, in the method 100 according to the invention, the generated training session 2 is formed of one or more blocks 10, 30, 70, each block consisting in turn of one or more sub-blocks or steps 12, 14, 15, ...; 31, 32, ...60; 71, 72.

Usefully, each sub-block is suitably defined as a function of at least one parameter related to one or more of said discrete reference values I_{M} of the waveform 1 and of a chosen reference physiological parameter related to a user or type of users who has/have to perform said at least one training session 2.

In particular, in a possible embodiment, in the phase 120 the intensity of training in each sub-block is determined using at least one of said parameters related to one or more of said discrete reference values I_{M} of the waveform 1 and the chosen reference physiological parameter.

In a possible embodiment, the chosen audio track comprises at least one piece of music.

Evidently, depending on the length of the training session to be generated, the audio track can comprise several pieces of music mixed together so as to reach the desired length of time.

According to a possible embodiment, the chosen audio track can comprise parts or even be made up of audio tracks which are not necessarily musical, for example motivational phrases.

Furthermore, in a possible embodiment, the chosen audio track and in particular its digital representation of a waveform representative of the chosen audio track, made up of the temporal sequence of the discrete reference values arranged in succession, may already be available and can be downloaded for example from a music streaming platform.

Alternatively, according to another possible embodiment, the method 100 according to the invention comprises a phase preceding the first phase 120, hereinafter indicated as pre-phase 110, in which, starting from an audio file comprising at least one chosen audio track and by means of said electronic processing unit, said digital representation of a waveform 1 representative of the chosen audio track is processed.

In both cases, whether the waveform 1 is generated in the pre-phase 110 or is already available, each discrete reference value I_{M} has a value that may vary between zero and one, and constitutes the value represented for each time unit, typically one second, in the waveform 1.

In the waveform illustrated in figure 2, said reference values are indicated with references I_{M1}, I_{M2}, ...I_{Mn} only for some of them and only for the sake of better illustrative clarity.

In a possible embodiment, the pre-phase 110 is carried out by Pulse Code Modulation (PCM).

In particular, for each defined sub-block, said reference physiological parameter comprises or consists of at least one of:
- a percentage of the cardiac threshold value or maximum cardiac frequency (CF) or heart rate (HR);
- a percentage of the value of the functional threshold power (FTP);
- a percentage of the maximum oxygen consumption value or VO2max (ml/min/kg).

Furthermore, according to a possible embodiment, the training session 2 is generated by setting a predefined reference level of difficulty, for example an intermediate level.

In the embodiment shown in figure 3, where the x-axis indicates the time and therefore the duration of the overall training session and its various parts, and the y-axis indicates a value in percentage of the reference parameter HR or FTP or VO2max, the generated training session 2 comprises three blocks, i.e. an initial warm-up block 10, a second central block 30 or training block, and a final cool-down block 70.

The initial block 10 comprises for example a first initial sub-block 12, a second initial sub-block 14, a third sub-block 16, a fourth sub-block 18, and a fifth sub-block 20.

In its turn, the central block 30 also comprises a plurality of sub-blocks defined in sequence and indicated with reference numbers from 31 to 60.

Finally, in the embodiment illustrated, the third block 70 comprises two blocks 71 and 72.

Evidently, it is possible that a block is formed by only one sub-block and in this case block and sub-block are one and the same.

In a possible embodiment, the pre-phase 110 comprises a first step 104 in which the audio track is sampled, at a pre-determined sampling frequency, for each playback channel of the audio file.

In accordance with this embodiment, the first phase 110 comprises a second step 108 in which, for each sampling time unit, typically one second, a reference value is calculated equal to the arithmetic mean among all the sample values sampled for all playback channels of the audio file in the corresponding sampling time unit.

The set of reference values calculated for each sampling time unit, arranged sequentially, results in the waveform which constitutes the digital representation of the audio track, such as the waveform 1 illustrated in figure 2.

For example, for a MP3 file with a standard quality level, e.g. for CD listening, there is a frequency of 44.1 kHz for each transmission channel and, therefore, for each second there are 44100 samples for each channel.

The electronic processing unit calculates, for every second, the reference values equal to the arithmetic mean among all the 44100 sample values sampled for all the channels.

Said reference value, which may vary between zero and one, constitutes the discrete reference value I_{M} represented for that second in the waveform 1.

In the waveform 1 illustrated in figure 2, said reference values are indicated with I_{M1}, I_{M2}, ...I_{Mn} only for some of them and only for the sake of better illustrative clarity.

In a possible embodiment, the phase 120 starts from the initial reference value I_{M1} of the waveform 1, previously available or generated, sequentially analyzing one by one the subsequent reference values I_{M2}, I_{M3}, ...I_{Mn}.

During the analysis, each time that along the generated waveform 1, starting from a reference value I_{Mn}, there is found for a predetermined time interval T1 a sequence of reference values I_{Mn} whose arithmetic mean differs from that of the current reference value I_{M} by a predetermined amount (in increase or decrease), then a new sub-block is generated and the sub-block preceding the start of the interval T1 is completed.

If the average of said values does not differ by at least a predetermined amount compared to I_{Mn}, then a further sub-block is not generated and the same verification is repeated starting from I_{Mn+1} for the same interval T1 (therefore scaled each time going forward, in other words towards the right along the x-axis).

For example, starting from the initial instant of the training session 2 in the generation phase, hence starting from the first reference value I_{M1}, the first sub-block is being generated.

In particular, regarding its duration (x-axis in figure 3), if proceeding along the waveform 1 from left to right, at a certain point, compared to a current reference value I_{Mn}, for example the sixtieth value I_{M60}, for a determined time interval T1 along the subsequent part of the waveform 1, there is found a sequence of reference values, e.g. I_{M61}, I_{M62}, etc., whose arithmetic mean differs from the current reference value I_{M60}, by said predefined amount, starting from the beginning of the interval T1, the first sub-block is completed and a new sub-block is generated, e.g. the second sub-block 14.

Said procedure is repeated in sequence until the end of the waveform 1 and every time the above-described condition occurs the sub-block under generation is completed (as regards the duration), and a successive one is opened.

The predetermined time T1 along the waveform 1 can be suitably chosen depending on the specific requirements and can, for example, vary between a few seconds, e.g. 10 seconds, and up to tens of seconds.

The predetermined amount can also be chosen suitably depending on the specific requirements, for example it can vary between 5% and 20% more or less of the preceding reference value to be taken into consideration.

In a possible embodiment, the method 100 according to the invention comprises an initial setting phase 105 where at least one between a minimum duration interval and a maximum duration interval is predefined for one or more of the sub-blocks of the training session 2 to be generated.

Usefully, in a possible embodiment of the method 100, the intervals of both minimum and maximum duration are defined.

In a possible embodiment, the minimum duration is the same for all the sub-blocks and can vary from a few seconds to tens of seconds, for example between 20 and 70 seconds; similarly, the maximum duration is also the same for all the sub-blocks and can be in the region of one or several hundred seconds, for example it can vary between 120 and 200 seconds.

The minimum and maximum duration of the various sub-blocks are chosen depending on the technical considerations of each training session.

For example, the minimum duration can be chosen so that a sub-block is not too brief and would therefore lose its importance in the context of the training session or the block of which it forms part, and/or that the number of sub-blocks is not too numerous so giving rise to excessive variations, etc.

In its turn, the maximum duration of the sub-blocks can be chosen so as not to be too long, so leading, for example, to a lengthy sub-block which might not be very stimulating or, on the contrary, may be excessively strenuous, and/or a few sub-blocks with a long duration leading to monotonous training sessions.

In particular, also in this embodiment, when the minimum duration interval has been predefined, in phase 120 the starting point is the initial reference value I_{M1} of the waveform 1 and the subsequent reference values I_{M2}, I_{M3}, ...I_{Mn}, etc. are analysed one by one sequentially.

During the analysis, when along the waveform 1, starting from a reference value I_{Mn} (which in that moment constitutes the current reference value), there is found for a predetermined time interval T1, a sequence of reference values I_{Mn} whose arithmetic average of values differs from said current reference value I_{M} by said predefined amount, a new sub-block is generated only if starting from the time when a previous sub-block (i.e., that being generated) has reached a duration at least equal to the predefined minimum duration interval. In this case, the reference value I_{Mn} to be considered for the subsequent sub-block coincides for example with the reference value I_{Mn} relative to the final instant of the minimum duration interval.

If the average of said values does not differ by at least one predetermined amount compared to I_{Mn}, so, similarly to what described above, a further sub-block is not generated and the same verification is repeated starting from I_{Mn+1} (which becomes the new current reference value) for the same interval T1 (scaled each time going forwards, i.e. towards the right along the x-axis, as schematically illustrated in figure 2 for two successive time intervals T1, the second of which is represented with a dashed line).

Furthermore, in this embodiment, when the maximum duration interval has been defined, in the phase 120, starting from the initial reference value I_{M1} of the waveform 1 and sequentially analyzing one by one the subsequent reference values I_{M2}, I_{M3}, ... I_{Mn}, every time that, along the generated waveform 1, once said maximum duration interval of a sub-block has elapsed, a sequence of reference values is not found whose arithmetic average, for a predetermined time interval T1, differs (in increase or decrease) from the current reference value I_{M} by a predefined amount, a new sub-block is generated starting from the time the previous sub-block (i.e. the one being generated) has reached a duration equal to the maximum duration interval. In this case, the reference value I_{Mn} to be considered initially as the current reference value for the successive sub-block coincides, for example, with the reference value I_{Mn} relative to the final instant of the maximum duration interval.

Also in this case, said procedure is repeated in sequence until the end of the waveform 1 and every time that the above condition occurs, the sub-block being generated is completed and the next one is opened.

As can be easily appreciated, in the method 100 according to the invention, the two previous procedures to generate sub-blocks, i.e. with and without predefinition of the minimum/maximum duration intervals, can both be available to the users and carried out at their discretion depending on specific requirements.

Similarly to what described above, also in this embodiment both the predetermined time T1 along the waveform 1 and the predetermined amount can be suitably chosen, depending on the specific requirements as mentioned above.

Usefully, in the method 100 according to the invention, the electronic processing unit also calculates the training intensity (i.e. the performance intensity of each sub-block) to be assigned along each sub-block, i.e. the value along the y-axis in figure 3 to be assigned gradually along each sub-block being generated.

In particular, the workload or training intensity of each sub-block depends on, and in particular is at least proportional to, the mean of the reference values I_{Mn} belonging to the same sub-block.

In particular, in the method 100 according to the invention, the training intensity or workload is calculated as a function of at least a percentage value of the reference physiological parameter used, whether it be the cardiac threshold HR, the functional threshold power FTP or the oxygen uptake VO2 max, defined for a corresponding sub-block, and of a value equal to the arithmetic mean of the reference values I_{M} of the waveform 1 belonging to the same sub-block.

Whichever parameter is used, the chosen value varies from a maximum value to a minimum value, with said limit values taking into account various factors, such as physiological limits of the user, sufficient training level, training objectives, etc., which substantially mean the level of the user, for example, beginner, intermediate, advanced.

Therefore, the effective range for a user will be between the minimum parameter value and a percentage of the maximum parameter value, scalable depending on his "level".

For example, the interval of the percentage of the cardiac threshold value (HR) can be selected for each sub-block from 50% to 100%, while for the functional threshold power (FTP) it can vary between 55% and 150%; if the physiological parameter selected is FTP, there is a reference range for the user which varies from 55% to 150% of his FTP. By setting a value equal to 60% for a beginner level, his effective working range varies from 55% to 90% of his FTP.

For example, the workload is calculated taking into consideration a suitable proportion within the respective range of reference values I_{Mn} (variable between 0 and 1) and the reference physiological parameter used (maximum workload and minimum workload).

In practice, the workload is for example proportional to the arithmetic mean of the reference values I_{Mn} of each sub-block and is expressed in a percentage scale which depends on the chosen reference physiological parameter (FTP, VO2max or HR max). In this way, the training workload values are relative to and equal for all users, with the advantage for the trainer that the preparation work for the session is reduced and simplified and can be "personalized" during each user's class, for example on the basis of measurement of the working parameters of the exercise machine.

For example, if the average of the reference values I_{M} in a sub-block is equal to 0.7, the workload for that sub-block can be calculated by using the following formula: Workload (for that sub-block) = Min workload + ((Max workload - Min workload) * Average I_{M}), which in the numeric example given above results in Workload = 55 + ((90 - 55) * 0.7) = 79,5% (which can be rounded up to 80%).

Therefore, for that sub-block, the workload will be equal to about 80% of the user's FTP. Evidently, said workload can be calculated in other ways and/or using other formulae.

Conveniently, according to a possible embodiment, the cadence of the machine used, or execution cadence of each sub-block, is calculated as a function of the beats per minute (BPM) of the portion of the audio track pertaining to the corresponding sub-block.

For example, said cadence for a bicycle is the pedalling cadence, for a treadmill it is the running cadence, for a rowing machine the rowing rhythm, etc.

In particular, if the number of the beats per minute in a sub-block is inferior to the predefined threshold value, the cadence for said sub-block is defined equal to the value of the beats per minute, and if the beats per minute in a sub-block are equal or superior to the predefined threshold value, the cadence for said sub-block is defined equal to half of the beats per minute.

Said values are also chosen in such a way that the various sub-blocks are executed with all due efficacy.

As previously noted, the training session 2 is generated by setting a predefined reference difficulty level, for example, an intermediate level, and usefully in the method according to the invention, said difficulty level is selectively adjustable and the adjustment to the pre-set level can be made before or after generation of the at least one training session 2, for example taking it to an inferior or superior level.

Once all the sub-blocks have been generated, a training session will be generated substantially automatically, such as the session 2 illustrated in figure 3.

Each session can be visualized directly on a monitor and/or saved in a memory for future use.

Furthermore, should it be desirable or necessary, the trainer has the opportunity to refine or better calibrate the generated session, or part of it, for example by acting on a graphical interface and "manually" modifying for example the duration of one or more of the automatically generated sub-blocks as previously described, and/ or the workload. Naturally, if the user is training on an indoor cycle, the user himself can manually modify the resistance until the correct percentage of the parameter used, e.g. FTP, appears on the display. If however the user is training on a bicycle with automatic resistance adjustment, the resistance will be adjusted automatically so as to obtain a power output equal to that percentage of FTP (also considering the pedalling cadence).

In practice, it has been demonstrated how the method 100 according to the invention reaches its intended aim in that it makes it possible to generate, almost automatically, a training session subdivided into various sub-blocks in which the intervention of the trainer is unnecessary or is required only minimally, for example to initially set up one or more desired parameters, and/or in order to refine the session generated.

It should be noted how in the method 100 according to the invention, in a totally innovative manner, each training session is generated starting from an audio track, and in particular from an audio file containing one or more preferred musical pieces.

Furthermore, as can be easily appreciated, the present invention can be implemented as a computer program product, for example it can be used with any electronic device, even of the portable type equipped with a suitable processor.

Therefore, the present invention also includes a computer program product recorded on at least one medium which can be read by an electronic processing unit, said software program including a plurality of instructions which, once carried out by said electronic processing unit, executes or causes the execution of a method 100 according to that described above.

Naturally, without prejudice to the scope of the invention, many variations and implementation details thereof may be applied to the above-described exemplary and non-limiting embodiments, without departing from the scope of protection of the invention as defined in the accompanying claims.

## Claims

1. Method (100) for generating at least one training session (2) to be performed using at least one exercise machine, **characterized in that** it comprises at least one phase (120) in which, starting from a digital representation of a waveform (1) which is representative of a chosen audio track and is constituted by a time sequence of discrete reference values (I_{M}), there is generated said at least one training session (2) formed of one or more blocks (10, 30, 70) each block consisting in turn of one or more sub-blocks (12, 14, 15,. 31, 32,...60; 71, 72), wherein each sub-block is defined as a function of at least one parameter related to one or more of said discrete reference values (I_{M}) of the waveform (1) and of a reference physiological parameter related to a user or type of users who has/have to perform said at least one training session (2).

2. Method (100) according to claim 1, wherein in said at least one phase (120) the training intensity of each sub-block is determined using at least one of said parameter relative to one or more of said discrete reference (I_{M}) values of waveform (1) and said reference physiological parameter.

3. Method (100) according to claim 1 or 2, wherein, for each defined sub-block, said reference physiological parameter comprises or consists of at least one of:
- a percentage of the cardiac threshold value or heart rate;
- a percentage of the value of the functional threshold power;
- a percentage of the maximum oxygen consumption value.

4. Method according to one or more of the previous claims, further comprising a pre-phase (110) in which there is generated, starting from an audio file comprising at least one chosen audio track, said digital representation of a waveform (1) which is representative of the chosen audio track.

5. Method (100) according to claim 4, wherein said pre-phase (110) comprises a first step (104) in which said audio track is sampled, at a predetermined sampling rate, for each playback channel of the audio file.

6. Method (100) according to claim 5, wherein said pre-phase (110) comprises a second step (108) in which, for each sampling time unit, there is calculated a reference value (I_{M}) having a value equal to the arithmetic mean among all sample values calculated for each playback channel in the corresponding sampling time unit, the set of discrete reference values (I_{M}) calculated sequentially for each sampling time unit forming said digital waveform (1).

7. Method (100) according to one or more of the preceding claims, wherein in said at least one phase (120), starting from the initial reference value (I_{M1}) of the waveform (1) and sequentially analyzing one by one the subsequent reference values (I_{M2}; I_{M3}; ... I_{Mn}), when, along said waveform (1), starting from a reference value (I_{Mn}), there is found for a predetermined time interval (T1) a sequence of reference values (I_{Mn+1}; I_{Mn+2}) whose arithmetic mean differs, in increase or decrease, from the reference value (I_{Mn}) by a predetermined amount, a new sub-block is generated.

8. Method (100) according to one or more of the preceding claims, comprising an initial phase (105) in which at least one of a minimum duration interval and a maximum duration interval is predefined for each sub-block of the training session (2) to be generated.

9. Method (100) according to claim 8, wherein in said initial phase (105) at least said interval of minimum duration is predetermined, and wherein in said at least one phase (120), starting from the initial reference value (I_{M1}) of the waveform (1) and sequentially analyzing one by one the subsequent reference values (I_{M2}; I_{M3}; ... I_{Mn}), even when along the waveform (1), starting from a reference value (I_{Mn}), there is found, for a predetermined time interval (T1), a sequence of reference values (I_{Mn}) whose arithmetic mean differs, in increase or decrease, from the reference value (I_{Mn}) by a predefined amount, a new sub-block is generated only from the moment when the previous sub-block has reached a duration equal to the predefined minimum duration interval.

10. Method (100) according to claim 8 or 9, wherein in said initial phase (105) at least said maximum duration interval is predefined, and wherein in said at least one pahse (120), starting from the initial reference value (I_{M1}) of the waveform (1) and sequentially analyzing one by one subsequent reference values (I_{M2}; I_{M3}; ... I_{Mn}), whenever, along the generated waveform (1), starting from a reference value (I_{Mn}), there is not found, for a predetermined time interval (T1), a sequence of reference values (I_{Mn}) whose arithmetic average of values differs, in increase or decrease, from the reference value (I_{Mn}) by a predetermined amount, a new sub-block is generated starting from the time when the previous sub-block has reached a duration equal to the maximum duration interval.

11. Method (100) according to one or more of the previous claims, in which the training intensity calculated for each sub-block is at least proportional to the mean of the arithmetic values (I_{M}) of the waveform (1) belonging to the same sub-block.

12. Method (100) according to one or more of the preceding claims in which the training intensity of each sub-block is calculated as a function of at least a percentage value of the reference physiological parameter used and of a value equal to the arithmetic mean of the reference values (I_{M}) of the waveform (1) belonging to the same sub-block.

13. Method (100) according to one or more of the preceding claims, wherein the execution cadence of each sub-block is calculated as a function of the beats per minute of the portion of the audio track pertaining to the corresponding sub-block.

14. A computer program product, which is recorded on at least one medium readable by a processing electronics unit, said computer program including a plurality of instructions which, when executed by said processing electronics unit, execute or cause the execution of a method (100) according to one or more of the preceding claims.
